# EUROPEAN PATENT SPECIFICATION

(11) **EP 3 368 141 B1**
(45) Date of publication and mention of the grant of the patent: **27.05.2020**
(21) Application number: 16860511.1
(22) Date of filing: 17.10.2016
(51) Int. Cl.: A61M 39/00, A61M 39/16, A61M 39/22

(54) **VENT ADAPTOR ASSEMBLIES AND METHODS OF MAKING THE SAME**
ENTLÜFTUNGSADAPTERANORDNUNGEN UND VERFAHREN ZUR HERSTELLUNG DAVON.
ENSEMBLES D'ADAPTATEUR D'ÉVENT ET PROCÉDÉ DE FABRICATION DE CEUX-CI.

(30) Priority: 30.10.2015 US 201562248505 P
(43) Date of publication of application: 05.09.2018
(73) Proprietor: C.R. Bard Inc., Murray Hill, NJ 07974 (US)
(72) Inventor: GLITHERO, Jason Iain, McDonough, Georgia 30252 (US); SWEENEY, Lyndsay Alison, Pine Lake, Georgia 30072 (US); PRIVITERA, Salvatore, Mason, Ohio 45040 (US)
(74) Representative: Hoffmann Eitle
(86) International application number: PCT/US2016/057348
(87) International publication number: WO 2017/074731

(56) References cited:
- WO-A1-93/21000
- US-A- 3 906 958
- US-A- 4 457 758
- US-A1- 2006 005 841
- US-A1- 2006 264 897
- US-A1- 2010 104 845
- US-A1- 2014 039 349
- US-A1- 2014 188 089
- US-A1- 2014 188 089

## Description

### BACKGROUND

Typically, urinary catheterization involves insertion of a urinary catheter (*e.g*., tube) through a patient's urethra and into the patient's bladder. Conventional drainage systems for urinary catheterization include a urinary catheter (*e.g.,* a Foley urinary catheter) coupled to a drainage bag using a drainage tube. As such, the urinary catheter allows the patient's urine to drain from the bladder through the drainage tube and into the drainage bag.

However, conventional drainage systems using urinary catheters may exhibit a number of fluid flow problems. For example, vapor lock, bends, or kinks in a drainage tube can restrict (*e.g*., prevent) the flow of urine from the urinary catheter to the drainage bag. Limiting the flow of urine may increase the patient's chances of developing a catheter-associated urinary tract infection ("CAUTI"), create a backpressure that increases patient discomfort (*e.g*., increase patient's urge to void bladder), and prevent accurate determination of urine output. Additionally, negative pressures may be created while using the drainage system (*e.g*., when rapidly draining urine from a bend in the drainage tube). Such negative pressures may inadvertently create a suction event within the bladder that may cause bladder mucosa of the bladder to be partially suctioned into the urinary catheter. Such suction events may damage the bladder mucosa which, in turn, may increase the patient's chances of developing a CAUTI. Typically, vents are coupled to a component of the drainage system to alleviate these drainage problems.

However, the vents may adversely affect the functionality of the drainage system. For example, conventional drainage system may be used to measure the intra-abdominal pressure of the patient. Typically, the intra-abdominal pressure is measured by clamping the drainage tube closed, filling the bladder with a solution (*e.g*., 0.9% sodium chloride solution) using a sample port, and measuring the mean pressure within the drainage tube. However, vents coupled to the drainage system may prevent accurate intra-abdominal pressure measurements.

Accordingly, manufacturers and users of urinary drainage bag systems, such as catheterization systems, continue to seek improvements thereto.

US 4 457 758 A discloses a venting assembly for a sealed body fluid drainage device.

US 2014/188089 A1 discloses a sintered porous polymeric caps and vents for components of medical devices.

US 2006/264897 A1 discloses an apparatus and method for delivering therapeutic and/or other agents to the inner ear and to other tissues.

US 3 906 958 A discloses a catheter adapter having filtered air vent.

### SUMMARY

In a first aspect of the present invention, there is disclosed a vent adaptor assembly according to claim 1.

In a second aspect of the present invention, there is disclosed a method according to claim 12.

Embodiments disclosed herein are directed to vent adaptor assemblies including a filter, methods of making such vent adaptor assemblies, methods of using such vent adaptor assemblies, and urinary drainage bag systems including such vent adaptor assemblies. The vent adaptor assemblies disclosed herein include a filter that is configured to permit gas (*e.g.,* air) to flow therethrough, while simultaneously at least partially preventing fluid present within the drainage bag system (*e.g*., urine) from flowing therethrough. In an embodiment, a vent adaptor assembly is disclosed. The vent adaptor assembly includes a drainage body defining a vent opening, a proximal opening, a distal opening spaced from the proximal opening, and a drainage lumen extending between the proximal and distal openings. The drainage lumen is in fluid communication with the vent opening. The vent adaptor assembly also includes a sintered porous polytetrafluoroethylene ("PTFE") filter disposed in fluid communication with the vent opening of the drainage body. The sintered porous PTFE filter defines a plurality of pores therein. The sintered porous PTFE filter is directly welded to a component of the vent adaptor assembly. The component is formed at least partially from polycarbonate. At least some of the plurality of pores of the sintered porous PTFE filter are at least partially occupied by the polycarbonate of the component of the vent adaptor.

In an embodiment, a vent adaptor assembly is disclosed. The vent adaptor assembly includes a drainage body defining a proximal opening, a distal opening, and a drainage lumen extending between the proximal and distal openings. The drainage body further at least partially defines a vent opening in fluid communication with the drainage lumen. The vent adaptor assembly also includes a filter in fluid communication with the vent opening of the drainage body. The vent adaptor assembly further includes a flow regulator. The flow regulator includes a flow barrier positioned at least partially within the drainage lumen of the drainage body. The flow regulator also includes an actuator operably coupled to the flow barrier. The actuator is configured to move the flow barrier between a first position and a second position. The flow barrier permits fluid to flow through the drainage lumen to the distal opening when the flow barrier is in the first position and substantially prevents the fluid to flow through the drainage lumen to the distal opening when the barrier is in the second position.

In an embodiment, a urinary drainage bag system is disclosed. The urinary drainage bag system includes a drainage bag having an inlet and configured to store at least one fluid therein. The urinary drainage bag system also includes a catheter. Additionally, the urinary drainage bag system includes at least one drainage tube fluidly coupling the inlet of the drainage bag to the catheter. The urinary drainage bag system further includes a vent adaptor coupled the at least one drainage tube between the drainage bag and the catheter. The vent adaptor assembly includes a drainage body defining a proximal opening, a distal opening, and a drainage lumen extending between the proximal and distal openings. The drainage body further at least partially defines a vent opening in fluid communication with the drainage lumen. The vent adaptor assembly also includes a filter in fluid communication with the vent opening of the drainage body. The filter includes a material that is at least substantially impermeable to a liquid and at least partially permeable to a gas. The vent adaptor includes a device that includes a cap configured to cover at least a portion of the vent opening or a flow regulator configured to regulate fluid flow through the drainage lumen the device. The device is configured to selectively switch between a first position and a second position. The device permits a flow of the gas through the filter into at least the entire drainage lumen when in the first position and restricts the flow of the gas through the filter into at least a portion of the drainage lumen when in the second position.

In an embodiment, a method of ultrasonically welding a sintered porous PTFE filter to a component of a vent adaptor assembly is disclosed. The method includes positioning the sintered porous PTFE filter adjacent to the component of the vent adaptor assembly. The sintered porous PTFE filter defining a plurality of pores therein and the component of the vent adaptor assembly at least partially formed of polycarbonate. The method also includes ultrasonically welding the sintered porous PTFE filter to the component of the vent adaptor assembly such that at least some of the polycarbonate of the component of the vent adaptor assembly softens and flows into and at least partially occupies some of the plurality of pores of the sintered porous PTFE filter.

In an embodiment, a method of using drainage bag system that includes a vent adaptor is disclosed. The method includes flowing a liquid through a drainage lumen defined by a drainage body of the vent adaptor. The method also includes flowing a gas through a vent opening that is at least partially defined by the drainage body, through a filter that is in fluid communication with the vent opening of the drainage body, and into at least an entirety of the drainage lumen of the drainage body. The method further includes, after flowing the gas through the vent opening, through the filter, and into at least an entirety of the drainage lumen; restricting the flow of the gas into at least a portion of the drainage lumen using a cap that is configured to at least partially cover the vent opening or a flow regulator that is configured to regulate fluid flow through the drainage lumen.

Features from any of the disclosed embodiments may be used in combination with one another, without limitation. In addition, other features and advantages of the present disclosure will become apparent to those of ordinary skill in the art through consideration of the following detailed description and the accompanying drawings.

### BRIEF DESCRIPTION OF THE DRAWINGS

The drawings illustrate several embodiments of the present disclosure, wherein identical reference numerals refer to identical or similar elements or features in different views or embodiments shown in the drawings.
**FIG. 1** is a top plan view of a urinary drainage bag system including a vent adaptor assembly, according to an embodiment.
**FIG. 2A** is an exploded isometric view of a vent adaptor assembly including a vent, according to an embodiment.
**FIG. 2B** is an assembled cross-sectional view of the vent adaptor assembly taken along line 2B-2B thereof.
**FIG. 3** is a flow diagram of a method of manufacturing the vent adaptor assembly shown in **FIGS. 2A-2B****,** according to an embodiment.
**FIG. 4A** is an exploded isometric view of a vent adaptor assembly including a filter and a sampling port, according to an embodiment.
**FIG. 4B** is an assembled cross-sectional view of the vent adaptor assembly shown in **FIG. 4A** taken along line 4B-4B thereof.
**FIG. 5A** is an exploded isometric view of a vent adaptor assembly including a vent adaptor and a sampling port adaptor, according to an embodiment.
**FIGS. 5B** and **5C** are isometric cutaway views of the assembled vent adaptor assembly shown in **FIG. 5A** illustrating a flow barrier thereof in first and second positions, according to an embodiment.
**FIG. 6** is an isometric cutaway view of a vent adaptor assembly including a vent and a flow regulator, according to an embodiment.
**FIG. 7** is an isometric view of a vent adaptor assembly including a vent, a flow regulator, and a sampling port, according to an embodiment.

### DETAILED DESCRIPTION

Embodiments disclosed herein are directed to vent adaptor assemblies including a filter, methods of making such vent adaptor assemblies, methods of using such vent adaptor assemblies, and urinary drainage bag systems including such vent adaptor assemblies. The vent adaptor assemblies disclosed herein include a filter that is configured to permit gas (*e.g.,* air) to flow therethrough, while simultaneously at least partially preventing fluid present within the vent adaptor assembly (*e.g*., urine) from flowing therethrough. In an embodiment, the filter includes a sintered porous PTFE filter, which may be in the form of a film that is ultrasonically directly welded to a polycarbonate component (*e.g*., non-porous polycarbonate component) of the vent adaptor assembly.

While many of the embodiments disclosed herein discuss the vent adaptor assemblies in relation to a urinary drainage bag system (*e.g*., a Foley catheterization system), it should be understood that the vent adaptor assemblies disclosed herein may be used in other types of drainage systems. For example, the vent adaptor assemblies disclosed herein may be used in a blood collection system or in other types of fluid drainage bag systems.

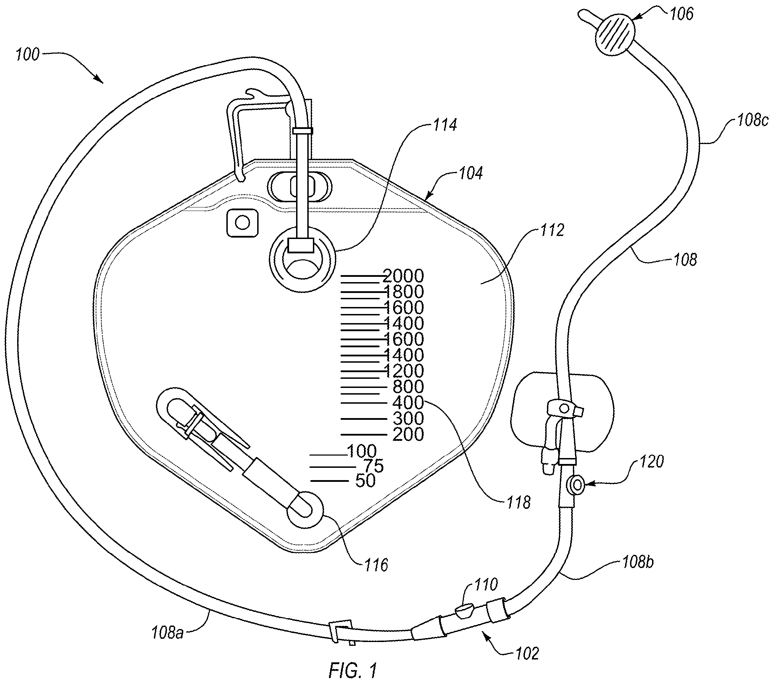

**FIG. 1** is a top plan view of a urinary drainage bag system 100 including a vent adaptor assembly 102, according to an embodiment. The urinary drainage bag system 100 illustrates one environment in which the vent adaptor assemblies disclosed herein may be employed. It should be noted that any of the vent adaptor assemblies disclosed herein may be incorporated in the urinary drainage bag system 100 or other suitable urinary drainage bag systems.

The urinary drainage bag system 100 includes a drainage bag 104 that is fluidly coupled to a catheter 106 (*e.g*., urinary catheter) via a drainage tube 108. When the catheter 106 is inserted into a bladder of a patient, a fluid (*e.g*., urine) may flow from and through the catheter 106 into the drainage bag 104. The vent adaptor assembly 102 may be physically and fluidly coupled to one or more components of the urinary drainage bag system 100 to improve fluid flow between the catheter 106 and the drainage bag 104. For example, the vent adaptor assembly 102 includes a vent 110 that permits air to enter the urinary drainage bag system 100, while partially or substantially preventing fluid (*e.g*., urine) from exiting the urinary drainage bag system 100 through the vent 110. The vent 110 also helps maintain atmospheric pressure within the urinary drainage bag system 100 at least at and/or near the vent 110. For example, the vent 110 may help maintain atmospheric pressure through substantially all of the drainage tube 108 and/or the urinary drainage bag system 100.

In the illustrated embodiment, the drainage bag 104 includes a front panel 112 and a back panel bonded together to form a fluid tight container. However, in other embodiments, the drainage bag 104 may include and/or may be formed by three or more panels, or a single body. Moreover, it should be appreciated that the panels of the drainage bag 104 may include flexible, rigid, resilient, any suitable material, or combinations of materials. In any event, the panels of the drainage bag 104 may be connected and/or bonded together in a manner that forms or defines an interior space of the drainage bag 104, which may contain fluid therein. Generally, the drainage bag 104 may have any suitable geometry. In the illustrated embodiment, the drainage bag 104 has a generally tear-shaped geometry. However, in other embodiments, the drainage bag 104 may have a generally circular geometry, a generally rectangular geometry, or other suitable geometry.

The drainage bag 104 also includes an inlet 114, which may be configured to accept fluid from the patient into the drainage bag 104. For example, the inlet 114 may receive or connect to the drainage tube 108 that may be in fluid communication with the catheter 106 positioned in the patient's bladder. In the illustrated embodiment, the catheter 106 may include a Foley urinary catheter, such that urine may flow into the drainage bag 104 from the catheter 106.

In an embodiment, the drainage bag 104 may include an outlet 116 (*e.g*., at or near a bottom of the drainage bag 104). For example, the outlet 116 may be configured to allow fluid collected in the drainage bag 104 to flow or drain from the drainage bag 104 (*e.g*., for collecting or extracting the fluid from the drainage bag 104). For example, the outlet 116 may include the Safety-Flow™ outlet device or another similar outlet device.

The front panel 112 of the drainage bag 104 may further include one or more graduated markings 118 that may indicate an amount of fluid collected in the drainage bag 104. For example, the graduated markings 118 may facilitate determining the amount of fluid discharged by the patient in a selected time span. In some embodiments, the graduated marking 118 may facilitate one or more determining or approximating time and/or date for draining, removing, or changing out the drainage bag 104. Flow problems within the drainage bag system 100 may prevent accurate measurement of the fluid using the graduated markings 118.

The drainage tube 108 may define a lumen therethrough that extends from the catheter 106 to the drainage bag 104. At least a portion of the drainage tube 108 may be sufficiently flexible to allow a clamp to at least partially collapse the drainage tube 108 such that fluid cannot pass through the collapsed portion of the drainage tube 108. Collapsing the drainage tube 108 may enable one or more tests to be performed, such as an intra-abdominal pressure measurement.

In some embodiments, the drainage tube 108 may include a first portion 108a and a second portion 108b. The first portion 108a may extend downstream from the vent adaptor assembly 102 at least partially towards the drainage bag 104. The second portion 108b may extend upstream from the vent adaptor assembly 102 at least partially towards the catheter 106. The drainage tube 108 may also include one or more additional portions, such as a third portion 108c. Alternatively, the vent adaptor assembly 102 may be integrally formed with the drainage tube 108.

In some embodiments, the urinary drainage bag system 100 may further include a sampling port 120. The sampling port 120 may be configured to permit a user *(e.g.,* a physician, a nurse, or a medical technician) to take a sample of fluid present within the drainage tube 108 in an at least substantially sterile and fluid tight manner. For example, the sampling port 120 may permit the user to take samples of the fluid using a syringe device, such as a catheter tip syringe or a syringe with needles. In operation, a tip (*e.g*., needle or catheter tip) of the syringe device may be inserted through the sampling port 120 such that the tip is in fluid communication with the fluid in the drainage tube 108. The sampling port 120 may reseal itself in an at least substantially fluid tight manner after the user takes the sample and removes the tip of the syringe device from the sampling port 120. In some embodiments, the sampling port 120 may be configured to permit the user to take multiple samples of the fluid from the drainage container. In some embodiments, the sampling port 120 may be configured to permit the user to inject a fluid into the drainage tube 108 (e.g., up into the bladder), such as during an intra-abdominal pressure measurement.

The sampling port 120 may be coupled to any component of the drainage bag system 100. For example, in the illustrated embodiment, the sampling port 120 is coupled to the second portion 108b of the drainage tube 108 such that the sampling port 120 is proximate to and upstream from the vent adaptor assembly 102. In such an embodiment, the second portion 108b may have a length sufficient to be collapsed by a clamp. The sampling port 120 may also be coupled to the third portion 108c of the drainage tube 108 such that the sampling port 120 is fluidly coupled to the catheter 106. Alternatively, the sampling port 120 may be coupled to a portion of the drainage tube 108 (*e.g*., the first portion 108a) that is downstream from the vent adaptor assembly 102. In some embodiments, the sampling port 120 may be omitted.

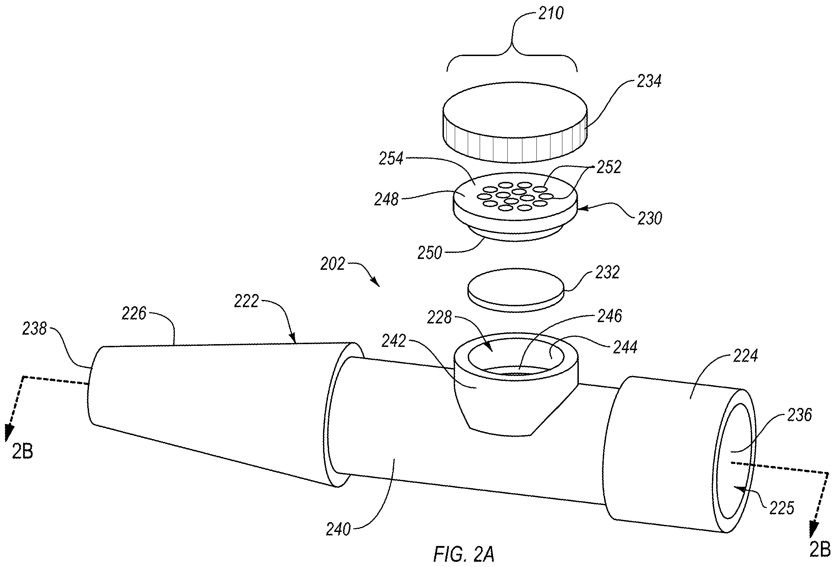

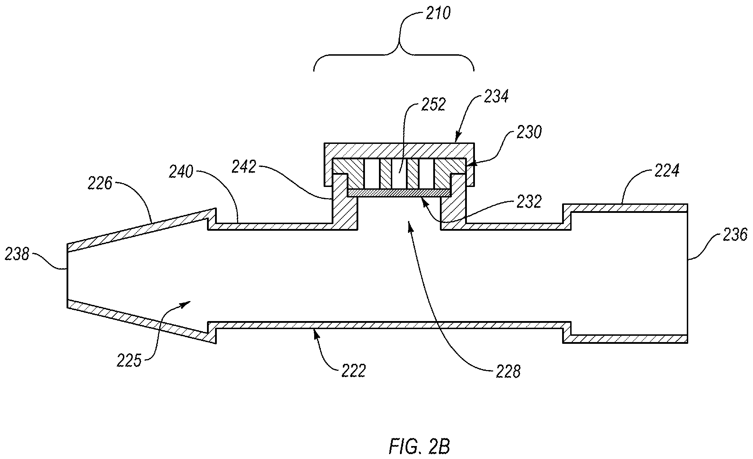

**FIGS. 2A** and **2B** are exploded isometric and assembled cross-sectional views, respectively, of a vent adaptor assembly 202 including a vent 210, according to an embodiment. The vent adaptor assembly 202 includes a drainage body 222 that includes a proximal portion 224 having a proximal opening 236, a distal portion 226 that is positioned downstream from the proximal portion 224 and having a distal opening 238, and an intermediate portion 240 that extends between the proximal portion 224 and the distal portion 226. The proximal portion 224 and the distal portion 226 are each configured to physically and/or fluidly couple the vent adaptor assembly 202 to a drainage tube (*e.g*., the drainage tube 108 shown **FIG. 1**).

In the illustrated embodiment, the proximal portion 224 of the drainage body 222 may be configured as a female connector that is configured to mate with a drainage tube (*e.g*., the drainage tube 108 shown **FIG. 1**). For example, an inner diameter of the proximal portion 224 at the proximal opening 236 may be sized to receive the drainage tube therein. In the illustrated embodiment, the distal portion 226 of the drainage body 222 may be configured as a male connector that is configured to mate with the drainage tube (*e.g*., the drainage tube 108 shown **FIG. 1**). For example, the outer diameter of the distal portion 226 at the distal opening 238 may be less than an inner diameter of the drainage tube. In an embodiment, the outer diameter of the distal portion 226 may be tapered and increase from the distal opening 238 towards the proximal portion 224 such that the outermost diameter of the distal portion 226, at a distance from the distal opening 238, is greater than the inner diameter of the drainage tube. In other embodiments, the proximal portion 224 may be configured as a male connector and/or the distal portion 226 may be configured as a female connector.

The drainage body 222 further defines a drainage lumen 225 extending through the intermediate portion 240, the proximal portion 224, and the distal portion 226. The drainage lumen 225 is in fluid communication with the proximal opening 236 and the distal opening 238 so that fluid can flow through the proximal opening 236, through the drainage lumen 225, and out of the distal opening 238. In the illustrated embodiment, the intermediate portion 240 may exhibit a substantially cylindrical shape. However, the intermediate portion 240 may exhibit any suitable shape.

The drainage body 222 also at least partially defines a vent opening 228 that is in fluid communication with the drainage lumen 225. In the illustrated embodiment, the vent opening 228 is at least partially defined by and located in the intermediate portion 240 of the drainage body 222. However, in other embodiments, the vent opening 228 may be located in the proximal portion 224 or the distal portion 226.

In the illustrated embodiment, the drainage body 222 includes a vent wall 242 that at least partially defines the vent opening 228. For example, the vent wall 242 exhibits a generally cylindrical shape that extends outwardly from the drainage body 222, such as extending outwardly from the intermediate portion 240 of the drainage body 222. However, the vent wall 242 may exhibit any suitable shape, such as a generally rectangular shape, a generally oval shape, or any other suitable shape. In an embodiment, the vent wall 242 may be omitted such that the filter 232 and/or the retention member 230 may be directly coupled to the intermediate portion 240 or other portion of the drainage body 222.

In an embodiment, the vent wall 242 may be integrally formed with the drainage body 222. In other embodiments, the vent wall 242 may be distinct and separate from the drainage body 222 and attached thereto using any suitable attachment method. For example, the vent wall 242 may be attached to the drainage body 222 by ultrasonic welding, an adhesive, a threaded connection, another suitable technique, or combinations thereof.

The vent wall 242 may include a first inner region 244 that exhibits a first inner diameter and a second inner region 246 that exhibits a second inner diameter that is less than the first inner diameter. The second inner region 246 may be located closer to the drainage lumen 225 than the first inner region 244. The decreased inner diameter of the second inner region 246 of the vent wall 242 provides a flange surface that supports, is attached to, and/or restricts movement of one or more components of the vent 210 (e.g., the filter 232, the retention member 230, etc.) to which the filter 232 may be attached and/or prevent the retention member 230. In an embodiment, the second inner region 246 of the vent wall 242 may be omitted such that the vent opening 228 exhibits a substantially uniform diameter.

The drainage body 222 may be formed from one or more different materials. For example, one or more components of the drainage body 222 (*e.g*., the proximal portion 224, the distal portion 226, the vent wall 242, or a component of the vent 210) may include one or more of a polymeric material, a metallic material, a ceramic, a composite, or another suitable material. In particular, the drainage body 222 may be at least partially formed from at least one of polycarbonate (*e.g*., non-porous polycarbonate), polyvinyl chloride ("PVC"), PTFE (*e.g*., sintered PTFE), low density polyethylene ("LDPE"), high density polyethylene ("HDPE"), polypropylene, polystyrene, polychlorotrifluoroethylene ("PCTFE"), aluminum, stainless steel, or any other suitable material.

The vent 210 includes at least one of a filter 232, a retention member 230, or a cap 234. The filter 232 is coupled to at least one of a portion of the drainage body 222 that defines the vent opening 228 or a portion of the vent 210 such as a retention member 230. The filter 232 is formed from a material that is configured to be at least partially permeable to gas (*e.g*., air), while being partially or substantially impermeable to a liquid (*e.g*., urine). The vent 210 is configured to at least substantially prevent liquid that is present within the drainage body 222 from exiting the through the vent opening 228. The vent 210 may further include a cap 234 configured to be removably attached to at least one of the drainage body 222 or the vent 210. The cap 234 exhibits a size and shape configured to cover the vent opening 228, thereby restricting gas from flowing into and out of the drainage lumen 225 through the vent opening 228 when the cap 234 is attached.

The filter 232 is formed and structured from a material that is substantially impermeable to a selected liquid (*e.g*., urine, water), while being at least partially permeable (*e.g.,* substantially permeable) to gas (*e.g.,* air). For example, the filter 232 may include a porous polymer film that defines a plurality of pores therein. The porous polymer film may be at least partially formed from PTFE, silicone, another hydrophobic material, or combinations thereof. For example, in an embodiment, the porous polymer film that is formed partially or substantially completed from a sintered porous PTFE film may be manufactured by sintering PTFE particles together to form the sintered film. The porous polymer film may be supported by (*e.g*., attached to) a porous substrate or the porous polymer film may be unsupported. In an embodiment, the substrate may facilitate attachment of the porous polymer film to at least one of the portion of the drainage body 222 that partially defines the vent opening 228 or a portion of the vent 210 (*e.g*., the vent wall 242 or the retention member 230).

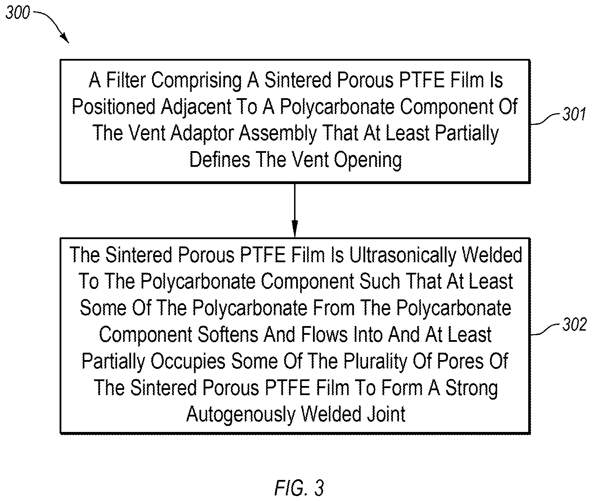

The filter 232 may be attached to at least one of the portion of the drainage body 222 that defines the vent opening 228 or a portion of the vent 210 in any suitable manner. In an embodiment, the filter 232 may be positioned between the retention member 230 and the second inner region 246 of the vent wall 242, while the retention member 230 is attached to the vent wall 242. As such, the retention member 230 and the second inner region 246 of the vent wall 242 prevent movement of the filter 232 and effectively secure the filter 232 within the vent opening 228 to the drainage body 222. In another embodiment, as will be discussed in more detail below with respect to **FIG. 3****,** the filter 232 may be welded (*e.g*., ultrasonically welded) to at least one the portion of the drainage body 222 that partially defines the vent opening 228 or a component of the vent 210 such as the retention member 230. In another embodiment, the filter 232 may be attached to at least one of the portion of the drainage body 222 that defines the vent opening 228 or a component of the vent 210 using an adhesive, a mechanical fastener, another suitable technique, or combinations thereof.

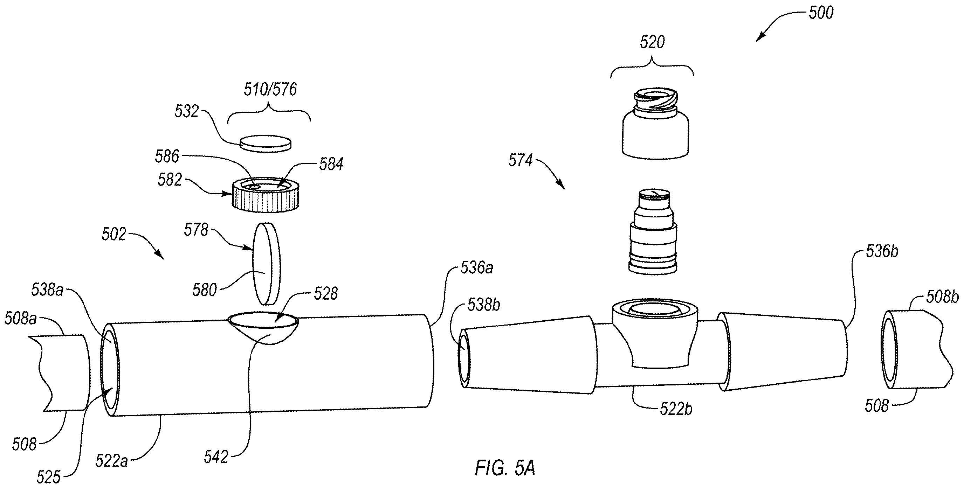

The retention member 230 may be configured to at least substantially cover the vent opening 228. For example, a portion of the retention member 230 may exhibit a shape and size that allows the portion of the retention member 230 to be positioned in the vent opening 228. The retention member 230 may be formed from any of materials disclosed herein for the drainage body 222. In an embodiment, the retention member 230 exhibits an at least two two-tiered structure including a top portion 248 and a bottom portion 250. The top portion 248 exhibits a generally disc-like geometry and exhibits a maximum diameter that is greater than the inner diameter of the first inner region 244 of the vent wall 242. The bottom portion 250 exhibits a shape (*e.g*., disc-like geometry) and size (*e.g*., maximum diameter) that is configured to be positioned inside the vent opening 228 adjacent to the first inner region 244 of the vent wall 242. For example, the bottom portion 250 may exhibit a shape and size that substantially corresponds to the shape and inner diameter of the first inner region 244 of the vent opening 228. However, the top portion 248 and the bottom portion 250 may exhibit any suitable shape other than a generally disc-like geometry, such as a generally rectangular geometry, a generally oval geometry, or other suitable geometry. Additionally, the top portion 248 and the bottom portion 250 may exhibit a shape that is different than the shape of the first inner region 244 of the vent wall 242 and/or each other. For example, the top portion 248 or the bottom portion 250 may include a recess (not shown) formed therein that is configured to receive the filter 232 therein (see embodiment shown in **FIGS. 5A-5C**). Finally, the retention member 230 may include one or more additional portions, or one of the top portion 248 or the bottom portion 250 may be omitted.

The retention member 230 may define one or more apertures 252 formed therein. Each of the one or more apertures 252 may extend from an uppermost surface 254 of the top portion 248 to a bottommost surface (not shown) of the bottom portion 250. The one or more apertures 252 exhibits a shape and size configured to allow air to flow therethrough, while preventing the filter 232 from being able to pass therethrough.

The retention member 230 may be configured to be secured to the vent wall 242. For example, the retention member 230 may be attached to the vent wall 242 by press-fitting at least a portion of the retention member 230 (*e.g.,* the bottom layer 250) with the vent wall 242. In other embodiments, the retention member 230 may be attached to the vent wall 242 using an adhesive, welding (*e.g*., ultrasonic welding), a threaded attachment, mechanical fasteners, any other suitable attachment method, or combinations thereof. In an embodiment, the retention member 230 may be secured to the vent wall 242 in a manner that at least substantially prevents fluid from flowing through a seam therebetween.

The cap 234 is at least substantially impermeable to a gas. As such, positioning the cap 234 over a portion of the vent opening 228 may at least restrict (*e.g*., at least substantially prevent) gas from flowing in and out of the vent opening 228. For example, the cap 234 may be configured to cover at least some of (*e.g.,* all of) the one or more apertures 252 of the retention member 230. The cap 234 may also cover some of (*e.g.,* all) of the seam between the retention member 230 and the vent wall 242. As such, the cap 234 may enable intra-abdominal pressure measurements or similar pressure sensitive test to be performed.

The cap 234 may be reversibly attached to the vent 210 in any suitable manner. For example, the cap 234 may be attached to a portion of the vent 210 (*e.g.,* the retention member 230) using a weak adhesive, a threaded attachment, or an interference fit or snap fit with the retention member 230 and/or the vent wall 242. In another embodiment, the cap 234 may include a magnet or a magnetically-attractable material, while at least a portion of the vent 210 and/or the drainage body 222 may include magnet or a magnetically-attractable material. As such, the cap 234 may be magnetically attached to the vent 210 and/or the drainage body 22.

**FIG.** 3 is a flow diagram of a method 300 of manufacturing the vent adaptor assembly 202 shown in **FIGS. 2A-2B****,** according to an embodiment. In act 301 of the method 300, a filter 232 comprising a sintered porous PTFE film may be positioned adjacent to a polycarbonate component (e.g., non-porous polycarbonate component) of the vent adaptor assembly 202 that at least partially defines the vent opening 228. The sintered porous PTFE film defines a plurality of pores therein. The sintered porous PTFE film may be attached to a porous substrate (e.g., supported) or may not be attached to a substrate. The polycarbonate component may include a portion of the vent wall 242, the retention member 230, another portion of the drainage body 222 that at least partially defines the vent opening 228, a portion of a flow regulator (*e.g*., vent/flow regulator 510, 576 shown in **FIGS. 5A-5C**), etc.

In act 302 of the method 300, the sintered porous PTFE film is ultrasonically welded to the polycarbonate component such that at least some of the polycarbonate from the polycarbonate component softens and flows into and at least partially occupies some of the plurality of pores of the sintered porous PTFE film to form a strong autogenously welded joint. Heat generated by absorption of the ultrasound energy at least partially softens the polycarbonate material (*e.g*., heats the polycarbonate material near, at, or above its glass transition temperature). Softening the polycarbonate material such that the polycarbonate material flows into the plurality of pores defined by the sintered porous PTFE film allows the sintered porous PTFE film and the polycarbonate material to be heated to lower temperatures than if the sintered porous PTFE film is softened and flows into a plurality of pores defined by the polycarbonate material. As such, the ultrasonic welding process of the act 302 reduces deformation and/or other physically and/or chemically damage to the sintered porous PTFE film during the welding process. The inventors currently believe that ultrasonically welding the sintered porous PTFE film to the polycarbonate material by softening the polycarbonate material is more effective than welding the sintered porous PTFE film to the polycarbonate material by softening the PTFE due to the hydrophobicity, non-reactivity, and low coefficient of friction of PTFE.

Ultrasonically welding the sintered porous PTFE film to the polycarbonate material may be performed at an ultrasound frequency for the ultrasound energy of about 10 kHz to about 60 kHz, a weld distance (*e.g.,* a distance a ultrasound horn of a ultrasonic welding machine moves in the direction of the pressure being applied during the application of ultrasound to the piece to be welded) of about 0.001 inch (0.03 mm) to about 0.01 inch (0.3 mm), a weld time (*e.g.,* an amount of time the ultrasonic welding machine applies the ultrasound energy to the piece to be welded) of about 0.075 seconds to about 0.3 seconds, a hold time (*e.g.,* an amount of time the ultrasonic welding machine applies pressure to the to the piece to be welded during the "cooling" portion of a welding cycle) of about 0.5 seconds to about 2 seconds, and a gauge pressure (*e.g*., the pressure the ultrasound horn of the ultrasonic welding machine applies to the piece to be welded) of about 5 psi (34 kPa) to about 20 psi (140 kPa). More particularly, ultrasonically welding the PTFE film to the
polycarbonate material may be performed at an ultrasound frequency for the ultrasound energy of about 25 kHz to about 50 kHz (*e.g*., about 30 kHz, about 32 kHz to about 45 kHz, about 40 kHz to about 42 kHz, or about 50 kHz), a weld distance of about 0.0025 inch (0.064 mm) to about 0.006 inch (0.15 mm) (*e.g*., about 0.003 inch (0.08 mm) to about 0.004 inch (0.1 mm), about 0.0035 inch (0.089 mm) to about 0.0045 inch (0.11 mm), about 0.0050 inch (0.13 mm), or about 0.0055 inch (0.14 mm)), a weld time of about 0.1 seconds to about 0.2 seconds (*e.g*., about 0.12 seconds to about 0.15 seconds, about 0.15 seconds to about 0.18 seconds, or about 0.12 seconds to about 0.14 seconds), a hold time of about 0.75 seconds to about 1.25 seconds (e.g., about 0.8 seconds to about 0.9 seconds, about 0.8 seconds to about 1 second, or about 1 second to about 1.2 seconds), and a gauge pressure of about 7.5 psi (52 kPa) to about 15 psi (100 kPa) (*e.g.,* about 8 psi (55 kPa) to about 9 psi (62 kPa), about 8 psi (55 kPa) to about 10 psi (69 kPa), about 10 psi (69 kPa) to about 13 psi (90 kPa), about 12 psi (83 kPa)). It should be noted that any combination of the foregoing values and/or ranges for the ultrasound energy, the weld distance, the weld time, the hold time, and the gauge pressure may be employed for ultrasonically welding the PTFE film to the polycarbonate material.

In other embodiments, the method 300 may be modified such that the filter 232 comprises a material other than a sintered porous PTFE film *(e.g.,* a silicone, a stretched PTFE film) and a component to which the filter 232 to bonded comprises a material other than a polycarbonate material (*e.g*., HDPE). Similarly, the method 300 may be modified to attach the filter 232 to the polycarbonate component using an attachment process other than ultrasonic welding (*e.g*., an adhesive).

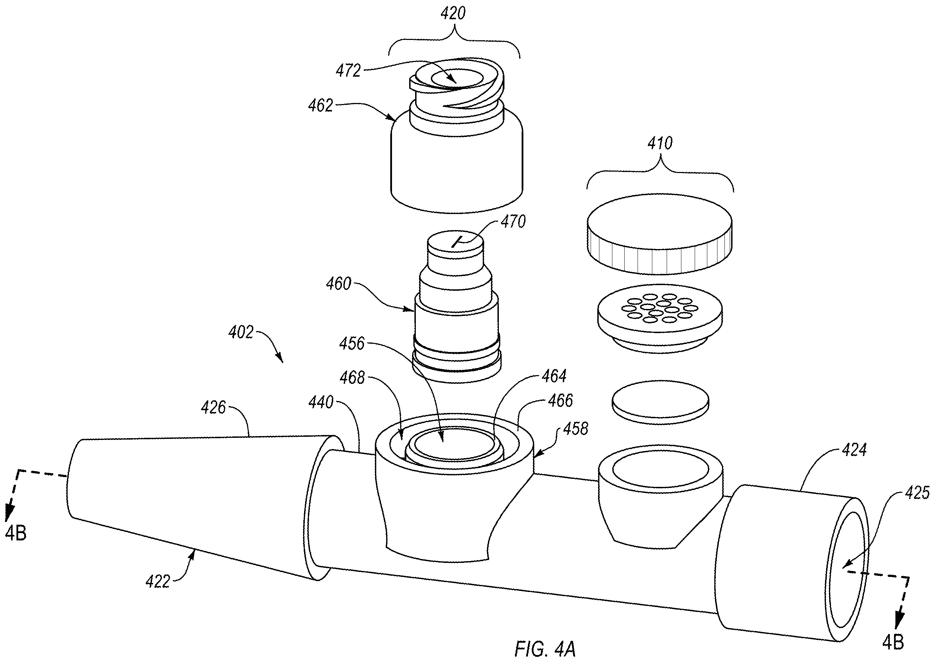

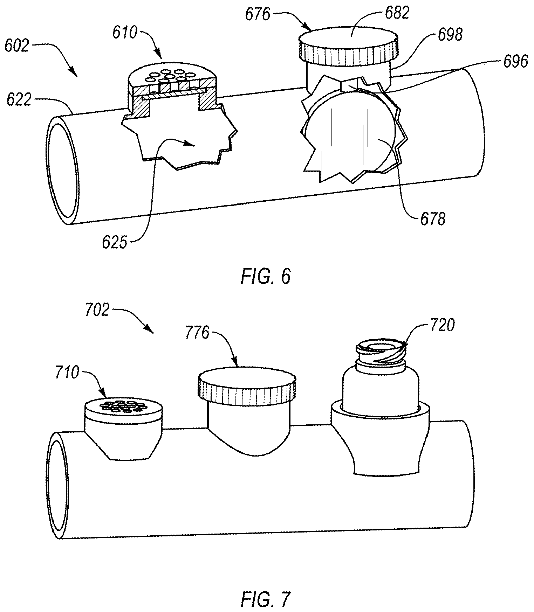

In some embodiments, the retention member 230 may be secured to the drainage body 222. For example the retention member 230 may be ultrasonically welded using the same or similar ultrasonic welding conditions discussed above for act 302, threadly fastened to, or otherwise attached to the vent wall 242. In some embodiments, one or more additional components of the vent adaptor assembly 202 may be attached to the drainage body 222. For example, a sampling port (*e.g*., sampling port 420 shown in **FIGS. 4A-4B**) and/or a flow regulator (*e.g*., flow regulator 676 shown in **FIG. 6**) may be attached to the vent adaptor assembly 202.

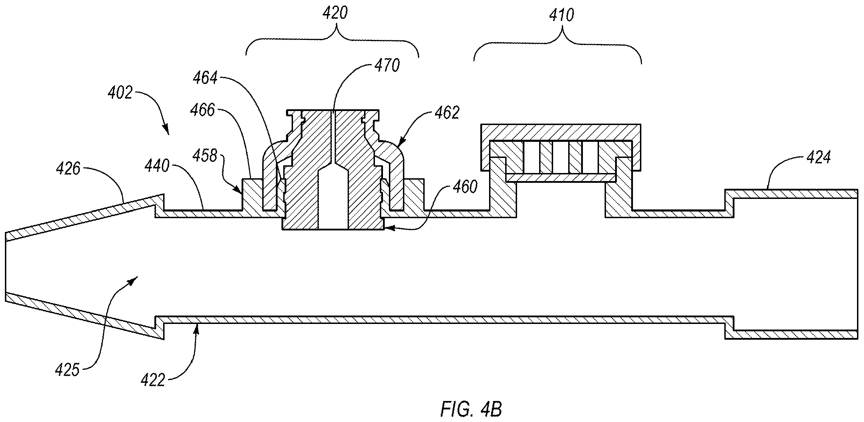

**FIGS. 4A** and **4B** are exploded isometric and assembled cross-sectional views, respectively, of a vent adaptor assembly 402 including a vent 410 and a sampling port 420, according to an embodiment. Features illustrated in and described in relation to **FIGS. 4A** and **4B** may be used in any of the embodiments disclosed herein. The vent adaptor assembly 402 is structurally similar to the vent adaptor assembly 202 shown in **FIGS. 2A-2B****.** Therefore, in the interest of brevity, an explanation of the components in both vent adaptors 202, 402 that are identical or similar to each other will not be repeated unless the components function differently in vent adaptor assemblies 202, 402.

The vent adaptor assembly 402 includes a sampling port 420. To accommodate the sampling port 420, an intermediate portion 440 of drainage body 422 partially defines a sampling opening 456 that provides access into a drainage lumen 425. However, in other embodiments, the sampling opening 456 may be formed in a proximal or distal portion 424, 426 of the drainage body 222. The sampling port 420 includes a sampling wall 458 extending from the drainage body 422 that also at least partially defines the sampling opening 456. The sampling port 420 further includes a sealing mechanism 460 that is configured to provide a user access to fluid positioned within the drainage body 422. Finally, the sampling port 420 includes a cover 462 that is configured to enclose a portion of the sealing mechanism 460.

In the illustrated embodiment, the sampling wall 458 includes an inner wall 464 and an outer wall 466 that extends about the inner wall 464. The inner wall 464 may partially define the sampling opening 456. An inner surface of the inner wall 464 may exhibit a size and shape configured to at least partially receive the sealing mechanism 460. For example, the inner surface of the inner wall 464 may exhibit a size and shape that substantially corresponds to a size and shape of a portion of the sealing mechanism 460 that is configured to be positioned in the sampling opening 456. In such an embodiment, the inner wall 464 and the sealing mechanism 460 may form a substantially fluid tight seal therebetween. The inner wall 464 and the second wall 466 may partially define a space 468 therebetween. The space 468 may be configured to receive a portion of the cover 462 therein. For example, an inner surface of the outer wall 466 may exhibit a size and shape that substantially corresponds to an outer surface of the cover 462 and/or an outer surface of the inner wall 464 may exhibit a size and shape that substantially corresponds to an inner surface of the cover 462. In such an embodiment, the inner wall 464 and/or the outer wall 466 may form an at least substantially fluid tight seal with the cover 462. In another embodiment, the sampling wall 458 may be substantially similar to the vent wall 242 shown in **FIGS. 2A-2B****.**

In an embodiment, the sampling wall 458 may be integrally formed with the drainage body 422. In other embodiments, the sampling wall 458 may be attached to the drainage body 422, for example, by ultrasonic welding, using an adhesive, a threaded attachment, or combinations thereof.

The sealing mechanism 460 may include any device that is configured to permit a user to take a sample of fluid present within the drainage lumen 425 in an at least substantially sterile and fluid tight manner. For example, the sealing mechanism 460 may include an access portion 470 (*e.g*., a slit) that may be configured to permit the user to take samples of the fluid using a syringe device, such as a needleless syringe (*e.g*., catheter tip syringe) or a syringe with needles. In operation, a tip of the syringe device (*e.g*., the catheter tip or needle) may be inserted through the access portion 470 of the sealing mechanism 460 such that the tip of the syringe device is in fluid communication with the fluid in the drainage lumen 425. As the sealing mechanism 460 may be formed from a suitable resilient material, the sealing mechanism 460 may reseal itself in an at least substantially fluid tight manner after the user takes the samples and removes the tip of the syringe device from the sealing mechanism 460. In some embodiments, the sealing mechanism 460 may be configured to permit the user to take multiple samples of the fluid from the drainage lumen 425. In some embodiments, the sealing mechanism 460 may permit the user to inject a fluid into the drainage lumen 425, such as during an intra-abdominal pressure measurement.

In an embodiment, the sealing mechanism 460 may be configured to be at least partially positioned within the sampling opening 456. In another embodiment, the sealing mechanism 460 may be positioned above the sampling opening 456. For example, the sealing mechanism 460 may be attached to the cover 462 and the cover 462 is configured to support the sealing mechanism 460 above the sampling opening 456.

As previously stated, the cover 462 is configured to at least partially enclose the sealing mechanism 460. For example, an inner surface of the cover 462 may exhibit a size and shape configured to enclose the sealing mechanism 460 at least partially therein. For example, at least a portion of the inner surface of the cover 462 may exhibit a size and shape that substantially corresponds to an outer surface of the sealing mechanism 460. In such an embodiment, the cover 462 and the sealing mechanism 460 may form an at least substantially fluid tight seal therebetween. The cover 462 may also define a cover hole 472 that permits access to the accessible portion 470 of the sampling mechanism 460 when the sampling port 420 is fully assembled. Finally, the cover 462 may be attached to the sampling wall 458 in any suitable manner (e.g., by welding, using an adhesive, press fitting, etc.).

In an embodiment, the sampling mechanism 460 and the cover 462 may be distinct components of the sampling port 420. In another embodiment, the sampling mechanism 460 and the cover 462 may be integrally formed. In another embodiment, the cover 462 may be omitted.

In the illustrated embodiment, the sampling port 420 is positioned downstream from a vent 410. However, in other embodiments, the sampling port 420 may be positioned upstream from the vent 410 or radially spaced from vent 410. The position of the sampling port 420 relative to the vent 410 may be more relevant when, for example, the vent adaptor assembly 402 includes a flow regulator (e.g., flow regulator 676 shown in **FIG. 6**) because the flow regulator may fluidly decouple the vent 410 from the sampling port 420 during an intra-abdominal pressure measurement, etc.

Configuring the vent adaptor assembly 402 to include both the vent 410 and the sampling port 420 improves the functionality of a urinary drainage bag system to which the vent adaptor assembly 402 is connected. For example, the vent adaptor assembly 402 eliminates the need for a separate drainage tube (*e.g*., the second portion 108b shown in **FIG. 1**) or other connections (*e.g*., the connection between the vent adaptor sub-assembly 502 and the sampling port adaptor 574 shown in **FIG. 5A-5C**) to fluidly couple the vent 410 and the sampling port 420 together. Eliminating the separate drainage tube and/or connections reduces the likelihood of leaks with a drainage assembly. Similarly, the vent adaptor assembly 402 simplifies the drainage assembly by reducing the number of components of the drainage bag system. Additionally, positioning the vent 410 proximate the sampling port 420 may improve fluid flow. For example, the vent adaptor assembly 402 eliminates kinks or bends in the drainage tube between the vent 410 and the sampling port 420 and better maintains a constant inner diameter of the lumen between the vent 410 and the sampling port 420.

**FIG. 5A** is an exploded isometric view of a vent adaptor assembly 500 including a vent adaptor sub-assembly 502 and a sampling port adaptor 574 according to an embodiment. Features illustrated in and described in relation to **FIG. 5A** may be used in any of the embodiments disclosed herein. The vent adaptor assembly 500 is structurally similar to the urinary drainage bag system 100 shown in **FIG. 1****.** Similarly, the vent adaptor sub-assembly 502 is structurally similar to the vent adaptor assembly 202, 402 shown in **FIGS. 2A-2B** and **4A-4B.** Therefore, in the interest of brevity, an explanation of the components in the urinary drainage bag system 100 and vent adaptor assembly 500 and the vent adaptor assemblies 202, 402, 502 that are identical or similar to each other will not be repeated unless the components function differently.

The vent adaptor assembly 500 includes a vent adaptor sub-assembly 502 coupled to a sampling port adaptor 574. Both the vent adaptor sub-assembly 502 and the sampling port adaptor 574 are fluidly coupled to a urinary drainage bag system via drainage tubes 508. For example, the vent adaptor sub-assembly 502 may be fluidly coupled to a first portion 508a of the drainage tube 508 and the sampling port adaptor 574 may be fluidly coupled to a second portion 508b of the drainage tube 508. In an embodiment, the sampling port adaptor 574 may be integrally formed with the vent adaptor sub-assembly 502 such that the vent adaptor sub-assembly 502 directly coupled together (as shown), or indirectly coupled together (*e.g*., via a second portion 108b of the drainage tube 108 of **FIG. 1**).

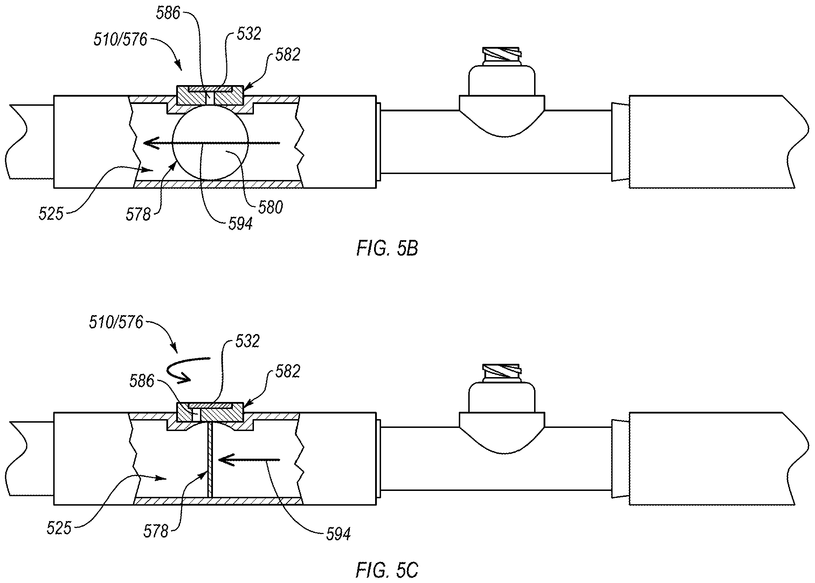

The vent adaptor sub-assembly 502 may include a vent 510 and a flow regulator 576 (collectively referred to as "vent/flow regulator 510, 576") and the sampling port adaptor 574 may include a sampling port 520. The vent adaptor sub-assembly 502 includes a first drainage body 522a that defines a first proximal opening 536a, a first distal opening 538a, and a drainage lumen 525 **(****FIGS. 5B-5C****)** extending between and in fluid communication with the first proximal opening 536a and the first distal opening 538a. The sampling port adaptor 574 includes a second drainage body 522b that defines a second proximal opening 536b and a second distal opening 538b. In an embodiment, the sampling port adaptor 574 may be integrally formed with the vent adaptor sub-assembly 502 such that the vent adaptor sub-assembly 502 includes a single drainage body that includes the vent/flow regulator 510, 576 and the sampling port 520.

The respective portions of the first drainage body 522a including the first proximal opening 536a and the first distal opening 538a are configured to be female connectors. The respective portions of the first drainage body 522b including the second proximal opening 536b and the second distal opening 538b are configured to be tapered male connectors. For example, the portion of the drainage body 522a including the first proximal opening 536a may be configured to receive and be coupled to the portion of the second drainage body 522b including the second distal opening 538b. It is understood that at least one of the first proximal opening 536a or the first distal opening 538a may be configured as part of a female connector and/or at least one of the second proximal opening 536b or the second distal opening 538b may be configured as part of a male connector.

The flow regulator 576 may include any suitable valve that is configured to regulate fluid flow through the drainage lumen 525. In an embodiment, the flow regulator 576 may include a flow barrier 578 that is configured to be at least partially disposed in the drainage lumen 525. The flow barrier 578 permits fluid to flow through the drainage lumen 525 when the flow barrier 578 is in a first position (*e.g*., as shown in **FIG. 5B**) and substantially prevents the fluid to flow through the drainage lumen 525 when the flow barrier 578 is in a second position (*e.g*., as shown in **FIG. 5C**). For example, the flow barrier 578 may include a face 580 that exhibits a disc-like shape. The face 580 may exhibit a size and peripheral shape that substantially corresponds to an inner surface of the first drainage body 522a when the flow barrier 578 is in the second position (*e.g.,* a butterfly valve). Alternatively, the flow barrier 578 may include a generally spherical geometry having a cutout formed therein (*e.g.,* a ball valve) or any other suitable flow barrier (*e.g.,* a check valve, a diaphragm valve, a gate valve, etc.). The flow barrier 578 may be movable between the first position and the second position.

The flow regulator 576 further includes an actuator 582 that is operably coupled to the flow barrier 578. The actuator 582 may be configured to move the flow barrier 578 between the first position and the second position and *vice versa.* For example, in the illustrated embodiment, the actuator 582 is directly coupled to the flow barrier 578. The actuator 582 may be coupled to the flow barrier 578 such that manipulating the actuator 582 (*e.g*., rotating the actuator 582) causes the flow barrier 578 to move (*e.g*., rotate) within the first drainage body 522. The actuator 582 may include a knob, a latch, a motor that operates in response to a signal, or any other suitable actuator. When the vent adaptor sub-assembly 502 is fully assembled, the actuator 582 may be at least partially positioned outside of the first drainage body 522a. The actuator 582 may be formed from any of the materials disclosed herein for the drainage body 222.

The vent 510 includes a filter 532 that is substantially similar to any of the filters disclosed herein, such as the filter 232. The filter 532 is coupled to the flow regulator 576 to form the vent/flow regulator 510, 576. For example, the actuator 582 may partially define a recess 584 that is configured to at least partially receive the filter 532 therein and the filter 532 may be attached to the actuator 582. For example, the filter 532 may be formed from a sintered porous PTFE film that is ultrasonically welded to a polycarbonate actuator 582. In another embodiment, the recess 584 may be omitted and the filter 532 may be coupled to a surface of the actuator 582. In another embodiment, the vent/flow regulator 510, 576 may further include a retention member (see **FIG. 2**) that is secured to the actuator 582. In such an embodiment, the filter 532 may be positioned between the actuator 582 and the retention member and the filter 532 may be attached to at least one of the actuator 582 or the retention member.

The vent 510 may further include a flow channel 586 that extends from the recess 584 through one or more components of the vent/flow regulator 510, 576 (*e.g.,* the actuator 582) to be in fluid communication with the drainage lumen 525 when the vent/flow regulator 510, 576 is fully assembled. For example, the one or more components of the vent/flow regulator 510, 576 may partially define the flow channel 586. As such, the flow channel 586 may fluidly couple the filter 532 to the drainage lumen 525 when the filter 532 is attached to the actuator 582. In the illustrated embodiment, the flow channel 586 extends completely through the actuator 582 **(****FIGS. 5B** and **5C****).** In an embodiment, the flow channel 586 may be offset from a center of the actuator 582 such that, when the flow barrier 578 is in the second position for obstructing/blocking the drainage lumen 525 **(****FIG. 5C****),** the flow channel 586 is only fluidly coupled to a portion of the drainage bag system 500 that is either upstream or downstream from the flow barrier 578. In another embodiment, the flow channel 586 may also extend partially through the flow barrier 578. In an embodiment, the flow channel 586 may extend from the recess 584 to the drainage lumen 525 such that the flow channel 586 is fluidly coupled to a portion of the drainage lumen 525 that is isolated from the sampling port adaptor 574 from the flow barrier 578 when the flow barrier 578 is in the second position for obstructing/blocking the drainage lumen 525 **(****FIG. 5C****).**

The vent adaptor sub-assembly 502 may include a vent opening 528. For example, the first drainage body 522a may partially define the vent opening 528. In an embodiment, the vent opening 528 may further be defined by a vent wall 542, or the vent wall 542 may be omitted. The vent opening 528 may be configured to have at least a portion of the vent/flow regulator 510, 576 positioned therein. In one embodiment, the vent/flow regulator 510, 576 may include the actuator 582 directly coupled to the flow barrier 578. As such, the vent opening 528 may exhibit a size and shape that allows to the actuator 582 to be directly coupled the flow barrier 578 when the vent adaptor sub-assembly 502 is fully assembled **(****FIGS. 5B** and **5C****).** For example, the vent wall 543 may not significantly extend outwardly from the rest of the first drainage body 522a thereby allowing the actuator 582 to be directly coupled to the flow barrier 578 when the vent adaptor sub-assembly 502 is fully assembled. In another embodiment, the vent/flow regulator 510, 576 may include the actuator 582 indirectly coupled to the flow barrier 578 **(****FIG. 6****).**

The vent opening 528 may also be configured to facilitate operation of the vent/flow regulator 510, 576. In an embodiment, the vent opening 528 may be sufficiently large to permit the flow channel 586 to be in fluid communication with the drainage lumen 525 through the vent opening 528 (e.g., not through the flow barrier 578 or another component of the vent adaptor sub-assembly 502). In another embodiment, the vent opening 528 may be configured to form an at least substantially liquid tight seal when the vent/flow regulator 510, 576 is positioned therein. For example, the vent opening 528 and the vent/flow regulator 510, 576 may be configured to permit the actuator 582 to rotate relative to the vent opening 528, while maintaining an at least substantially fluid tight seal between the vent wall 543 and the actuator 582.

**FIGS. 5B** and **5C** are isometric cutaway views of the assembled vent adaptor assembly 500 shown in **FIG. 5A** illustrating the flow barrier 578 in the first and second positions, according to an embodiment. Referring to **FIG. 5B****,** the vent/flow regulator 510, 576 may be positioned such that the flow barrier 578 is in the first position. In the first position, the flow barrier 578 permits fluid to flow around the flow barrier 578 and through the drainage lumen 525 to the first distal opening 538a. For example, the flow barrier 578 may exhibit a generally disc-like geometry having generally circular face 580. In such an embodiment, in the first position, the face 580 of the flow barrier 578 is substantially parallel to the flow of the fluid 594 or the face 580 may form an oblique angle relative to the flow of the fluid 594. When the flow barrier 578 is in the first position, the filter 532 may be in fluid communication with portions of the drainage lumen 525 upstream and downstream from the flow barrier 578. The portions of the drainage lumen 525 that are in fluid communication with the filter 532 may exhibit about atmospheric pressure.

Referring to **FIG. 5C****,** a user of the vent adaptor sub-assembly 502 may manipulate (*e.g*., rotate) the actuator 582 such that the flow barrier 578 is moved to the second position for obstructing/blocking the drainage lumen 525. In the second position, the flow barrier 578 substantially prevents the fluid to flow through the drainage lumen 525 to the first distal opening 538a. For example, when the flow barrier 578 exhibits a generally disc-like geometry, in the second position, the face 580 of the flow barrier 578 is substantially orthogonal to the flow of the fluid 594 and/or substantially corresponds to an interior geometry of the drainage lumen 525 to obstruct and substantially prevent fluid in the drainage lumen 525 from flowing downstream past the flow barrier 578 to the distal opening 538a. Additionally, when the flow barrier 578 is in the second position, the filter 532 may not be in fluid communication with portions of the drainage lumen 525 that are upstream or downstream from the flow barrier 578 depending on which side of the flow barrier 578 the flow channel 586 is located.

In an embodiment, the vent/flow regulator 510, 576 may be used to facilitate an intra-abdominal pressure measurement. For example, a user may manipulate the actuator 582 to move the flow barrier 578 to the second position. When the flow barrier 578 is in the second position, the flow barrier 578 may at least substantially prevent fluid present in the drainage lumen 525 from flowing through the drainage lumen 525 to the first distal opening 538a. The flow barrier 578 may also at least substantially prevent gas from flowing through the filter 532 to a portion of the drainage bag system 500 that is upstream from the flow barrier 578. The user may then insert fluid (*e.g.,* a saline solution) into the portion of the drainage bag system 500 that is upstream from the flow barrier 578 using, for example, the sampling port 520 or another device attached to the drainage bag system 500 (*e.g.,* another sampling port). A transducer (not shown) may also be coupled to the portion of the drainage bag system 500 that is upstream from the flow barrier 578 such that the transducer may measure the pressure within the portion of the drainage bag system 500 that is upstream from the flow barrier 578. For example, the transducer may be coupled to the portion of the drainage bag system 500 that is upstream from the flow barrier 578 using the sampling port 520 or another device coupled to the drainage bag system 500 (*e.g.,* another sampling port). The flow barrier 578 enables the transducer to accurately measure a pressure in the portion of the drainage bag system 500 that is upstream from the flow barrier 578 by at least substantially preventing the fluid from flowing downstream from the flow barrier 578 and preventing gas from flowing through the filter 532 and upstream from the flow barrier 578.

**FIG. 6** is an isometric cutaway view of a vent adaptor assembly 602 that includes a vent 610 and a flow regulator 676, according to an embodiment. Features illustrated in and described in relation to **FIG. 6** may be used in any of the embodiments disclosed herein. The vent adaptor assembly 602 is structurally similar to the vent adaptor assemblies 202, 402, and/or 502 shown in **FIGS. 2A-2B****,** **4A-4B****,** and **5A-5C****.** Therefore, in the interest of brevity, an explanation of the vent adaptor assemblies 202, 402, 502, and 602 that are identical or similar to each other will not be repeated unless the components function differently.

The vent adaptor assembly 602 includes a vent 610 that is not integrally formed with the flow regulator 676. For example, the vent 610 may be configured the same or similar to the vent 210 shown in **FIGS. 2A-2B****.** The vent 610 is spaced from the flow regulator 676. For example, in the illustrated embodiment, the vent 610 is positioned downstream from the flow regulator 676. The vent 610 may be positioned downstream from the flow regulator 676 when it is desirable to selectively isolate the vent 610 from a portion of the drainage assembly that is upstream from the flow regulator 676 (*e.g.,* for intra-abdominal pressure measurements). In other embodiments, the vent 610 may be positioned upstream from the flow regulator 676 if it is desirable to selectively isolate the vent 610 from a portion of drainage bag system that is downstream from the flow regulator 676 (e.g., when replacing a drainage bag).

The flow regulator 676 may include an actuator 682 indirectly coupled to a flow barrier 678. For example, the vent adaptor assembly 602 may include a drainage body 622 that defines a drainage lumen 625 and has a flow regulator wall 698 extending therefrom. In an embodiment, the flow regulator wall 698 may extend from the drainage body 622 and space the actuator 682 from the flow barrier 678. As such, the flow regulator 676 may include an elongated member 696 that extends between and is coupled to the actuator 682 and the flow barrier 678. The elongated member 696 may be configured to move the flow barrier 678 from a first position that allows fluid flow through the drainage lumen 625 to a second position that obstructs/blocks the drainage lumen 625, or *vice versa,* when the actuator 682 is manipulated. For example, the flow barrier 678 may be structured and function similar to or the same as the flow barrier 578 shown in **FIGS. 5A-5C****.** In an embodiment, the drainage body 622, the flow regulator wall 698, and the actuator 682 may form an at least substantially fluid tight seal therebetween.

**FIG. 7** is an isometric view of a vent adaptor assembly 702 that includes a vent 710, a flow regulator 776, and a sampling port 720, according to an embodiment. Features illustrated in and described in relation to **FIG. 7** may be used in any of the embodiments disclosed herein. The vent adaptor assembly 702 is structurally similar to the vent adaptors assemblies 202, 402, 502 and/or 602 shown in **FIGS. 2A-2B****,** **4A-4B****,** **5A-5C****,** and **6****.** Therefore, in the interest of brevity, an explanation of the vent adaptor assemblies 202, 402, 502, 602, and 702 that are identical or similar to each other will not be repeated unless the components function differently. For example, the vent 710 may be configured the same or similar to the vent 610 shown in **FIG. 6****,** the flow regulator 776 may be configured the same or similar to the flow regulator 676 shown in **FIG. 6****,** and the sample port 720 may be configured the same or similar to the sample port 420 shown in **FIGS. 4A-4B****.**

The flow regulator 776 may be positioned between the vent 710 and the sampling port 720. As such, the flow regulator 776 may isolate the vent 710 from the sampling port 720. The vent 710 may be positioned downstream from the flow regulator 776 when it is desired to isolate the vent 710 from a portion of the drainage bag system that is upstream from the flow regulator 776 (e.g., during an intra-abdominal pressure measurement). However, the vent 710 and the sampling port 720 may have any position relative to the flow regulator 776. For example, the vent 710 and the sampling port 720 may be positioned upstream from the flow regulator 776 to prevent patient discomfort from back pressure caused by moving the flow barrier (not shown) into the second position while simultaneously allowing a user to take a sample of a fluid present within the vent adaptor assembly 702.

While various aspects and embodiments have been disclosed herein, other aspects and embodiments are contemplated. The various aspects and embodiment disclosed herein are for purposes of illustration and are not intended to be limiting.

## Claims

1. A vent adaptor assembly (102), comprising:
a drainage body (222) defining a vent opening (228), a proximal opening (236), a distal opening (238) spaced from the proximal opening (236), and a drainage lumen (225) extending between the proximal (236) and distal openings (238), the drainage lumen being in fluid communication with the vent opening (228); and
a sintered porous polytetrafluoroethylene filter (232) disposed in fluid communication with the vent opening (228) of the drainage body, the sintered porous polytetrafluoroethylene filter (232) defining a plurality of pores therein,
the sintered porous polytetrafluoroethylene filter (232) being directly welded to a component of the vent adaptor assembly (102), the component formed at least partially from polycarbonate;
wherein at least some of the plurality of pores of the sintered porous polytetrafluoroethylene filter (232) are at least partially occupied by the polycarbonate of the component of the vent adaptor assembly (102).

2. The vent adaptor assembly of claim 1 wherein the component of the vent adaptor assembly includes the drainage body (222), optionally wherein the drainage body (222) includes a sampling port in fluid communication with the drainage lumen (225).

3. The vent adaptor assembly of claim 1 wherein the component of the vent adaptor assembly includes a retention member (230) that defines one or more apertures (252) therein, the one or more apertures (252) are fluidly coupled to the sintered porous polytetrafluoroethylene filter (232).

4. The vent adaptor assembly of claim 1, further comprising a flow regulator (512) disposed at least partially in the drainage lumen (225) of the drainage body (222), the flow regulator (512) configured to regulate fluid flow through the drainage lumen (225) to the distal opening (238).

5. The vent adaptor assembly of claim 4, wherein the flow regulator includes:
a flow barrier (578) positioned at least partially within the drainage lumen (225); and
an actuator (582) operably coupled to the flow barrier (578), the actuator configured to move the flow barrier (582) between a first position and a second position;
wherein the flow barrier permits fluid to flow through the drainage lumen (225) to the distal opening when the flow barrier (578) is in the first position and substantially prevents the fluid to flow through the drainage lumen (225) to the distal opening when the flow barrier (578) is in the second position wherein optionally the sintered porous polytetrafluoroethylene filter (232) is attached to the actuator (582), the actuator defines at least one flow channel (586) therein that is coupled to the sintered porous polytetrafluoroethylene filter.

6. The vent adaptor assembly of claim 1, further comprising a cap (234) configured to be removably attached to the drainage body (222) and exhibiting a size and shape configured to cover at least a portion of the vent opening (228).

7. The vent adaptor assembly of claim 1 wherein the sintered porous polytetrafluoroethylene filter (232) includes a sintered porous polytetrafluoroethylene film.

8. A urinary drainage bag system (100), comprising:
a drainage bag (104) including an inlet (114) and configured to store at least one fluid therein;
a catheter (106);
at least one drainage tube (108) fluidly coupling the inlet (114) of the drainage bag (104) to the catheter (106); and
the vent adaptor assembly (102) of any of claims 1-7, the vent adaptor assembly coupled to the at least one drainage tube (108) between the drainage bag (104) and the catheter (106).

9. The urinary drainage bag system of claim 8, wherein the at least one drainage tube includes:
a first drainage tube (108a) extending from the inlet (114) of the drainage bag to the vent adaptor assembly (102); and
a second drainage tube (108b) extending from the vent adaptor towards the catheter.

10. The urinary drainage bag system of claim 8 comprising the vent adaptor assembly of claim 5, wherein the flow barrier is positioned between a portion of the drainage lumen that is in fluid communication with the filter and the sampling port when the flow barrier is in the second position.

11. The urinary drainage bag system of claim 8, wherein the sampling port is positioned upstream from the flow regulator.

12. A method of ultrasonically welding a sintered porous polytetrafluoroethylene filter (232) to a component of a vent adaptor assembly (102), the method comprising:
positioning the sintered porous polytetrafluoroethylene filter (232) adjacent to the component of the vent adaptor assembly (102), the sintered porous polytetrafluoroethylene filter (232) defining a plurality of pores therein and the component of the vent adaptor assembly at least partially formed of polycarbonate; and
ultrasonically welding the sintered porous polytetrafluoroethylene filter (232) to the component of the vent adaptor assembly such that at least some of the polycarbonate of the component of the vent adaptor assembly softens and flows into and at least partially occupies some of the plurality of pores of the sintered porous polytetrafluoroethylene filter.

13. The method of claim 12, wherein ultrasonically welding the sintered porous polytetrafluoroethylene filter (232) to the component includes ultrasonically welding the sintered porous polytetrafluoroethylene filter to the component at a frequency of about 10 kHz to about 60 kHz and/or at a weld distance of about 0.001 inch (0.03mm) to about 0.01 inch (0.3mm) and/or at a weld time of about 0.075 seconds to about 0.3 seconds and/or at a hold time of about 0.5 seconds to about 2 seconds and/or at a gauge pressure of about 5 psi (34kPa) to about 20 psi (140kPa).

14. The method of claim 12, wherein the component includes non-porous polycarbonate component.

## Patentansprüche

1. Entlüftungsadaptermontage (102), umfassend:
einen Drainagekörper (222), der eine Entlüftungsöffnung (228), eine proximale Öffnung (236), eine von der proximalen Öffnung (236) beabstandete distale Öffnung (238) und ein Drainagelumen (225), das sich zwischen der proximalen (236) und der distalen Öffnung (238) erstreckt, definiert, wobei das Drainagelumen mit der Entlüftungsöffnung (228) in Fluidverbindung steht; und
einen gesinterten porösen Polytetrafluorethylenfilter (232), der in Fluidverbindung mit der Entlüftungsöffnung (228) des Drainagekörpers angeordnet ist, wobei der gesinterte poröse Polytetrafluorethylenfilter (232) eine Vielzahl von Poren darin definiert,
wobei der gesinterte poröse Polytetrafluorethylenfilter (232) direkt mit einem Bauteil der Entlüftungsadaptermontage (102) verschweißt ist, wobei das Bauteil mindestens teilweise aus Polycarbonat gebildet ist;
wobei mindestens einige der Vielzahl von Poren des gesinterten porösen Polytetrafluorethylenfilters (232) mindestens teilweise von dem Polycarbonat des Bauteils der Entlüftungsadaptermontage (102) eingenommen werden.

2. Entlüftungsadaptermontage nach Anspruch 1, wobei das Bauteil der Entlüftungsadaptermontage den Drainagekörper (222) beinhaltet, wobei der Drainagekörper (222) wahlweise einen Probenentnahmeport, der mit dem Drainagelumen (225) in Fluidverbindung steht, beinhaltet.

3. Entlüftungsadaptermontage nach Anspruch 1, wobei das Bauteil der Entlüftungsadaptermontage ein Rückhalteelement (230) beinhaltet, das eine oder mehrere Löcher (252) darin definiert, wobei das eine oder die mehreren Löcher (252) mit dem gesinterten porösen Polytetrafluorethylenfilter (232) fluidisch gekoppelt sind.

4. Entlüftungsadaptermontage nach Anspruch 1, weiter umfassend einen Durchflussregler (512), der mindestens teilweise in dem Drainagelumen (225) des Drainagekörpers (222) angeordnet ist, wobei der Durchflussregler (512) konfiguriert ist, um die Fluidströmung durch das Drainagelumen (225) zu der distalen Öffnung (238) zu regeln.

5. Entlüftungsadaptermontage nach Anspruch 4, wobei der Durchflussregler Folgendes beinhaltet:
eine Strömungsbarriere (578), die mindestens teilweise innerhalb des Drainagelumens (225) positioniert ist; und
einen Aktuator (582), der mit der Strömungsbarriere (578) betriebsbereit gekoppelt ist, wobei der Aktuator konfiguriert ist, um die Strömungsbarriere (582) zwischen einer ersten Position und einer zweiten Position zu bewegen;
wobei die Strömungsbarriere erlaubt, dass Fluid durch das Drainagelumen (225) zu der distalen Öffnung strömt, wenn sich die Strömungsbarriere (578) in der ersten Position befindet, und im Wesentlichen verhindert, dass das Fluid durch das Drainagelumen (225) zu der distalen Öffnung strömt, wenn sich die Strömungsbarriere (578) in der zweiten Position befindet, wobei der gesinterte poröse Polytetrafluorethylenfilter (232) wahlweise an dem Aktuator (582) befestigt ist, wobei der Aktuator mindestens einen Strömungskanal (586) darin definiert, der mit dem gesinterten porösen Polytetrafluorethylenfilter gekoppelt ist.

6. Entlüftungsadaptermontage nach Anspruch 1, weiter umfassend eine Kappe (234), die konfiguriert ist, um abnehmbar an dem Drainagekörper (222) befestigt zu sein, und eine Größe und Form vorweist, die konfiguriert sind, um mindestens einen Abschnitt der Entlüftungsöffnung (228) zu bedecken.

7. Entlüftungsadaptermontage nach Anspruch 1, wobei der gesinterte poröse Polytetrafluorethylenfilter (232) eine gesinterte poröse Polytetrafluorethylenfolie beinhaltet.

8. Urindrainagebeutelsystem (100), umfassend:
einen Drainagebeutel (104), der einen Einlass (114) beinhaltet und konfiguriert ist, um mindestens ein Fluid darin zu speichern;
einen Katheter (106);
mindestens einen Drainageschlauch (108), der den Einlass (114) des Drainagebeutels (104) mit dem Katheter (106) fluidisch koppelt; und
die Entlüftungsadaptermontage (102) nach einem der Ansprüche 1-7, wobei die Entlüftungsadaptermontage mit dem mindestens einen Drainageschlauch (108) zwischen dem Drainagebeutel (104) und dem Katheter (106) gekoppelt ist.

9. Urindrainagebeutelsystem nach Anspruch 8, wobei der mindestens eine Drainageschlauch Folgendes beinhaltet:
einen ersten Drainageschlauch (108a), der sich von dem Einlass (114) des Drainagebeutels zu der Entlüftungsadaptermontage (102) erstreckt; und
einen zweiten Drainageschlauch (108b), der sich von dem Entlüftungsadapter in Richtung des Katheters erstreckt.

10. Urindrainagebeutelsystem nach Anspruch 8, umfassend die Entlüftungsadaptermontage nach Anspruch 5, wobei die Strömungsbarriere zwischen einem Abschnitt des Drainagelumens, der mit dem Filter in Fluidverbindung steht, und dem Probenentnahmeport positioniert ist, wenn sich die Strömungsbarriere in der zweiten Position befindet.

11. Urindrainagebeutelsystem nach Anspruch 8, wobei der Probenentnahmeport dem Durchflussregler vorgeschaltet positioniert ist.

12. Verfahren zum Ultraschallverschweißen eines gesinterten porösen Polytetrafluorethylenfilters (232) mit einem Bauteil einer Entlüftungsadaptermontage (102), wobei das Verfahren Folgendes umfasst:
Positionieren des gesinterten porösen Polytetrafluorethylenfilters (232) angrenzend an das Bauteil der Entlüftungsadaptermontage (102), wobei der gesinterte poröse Polytetrafluorethylenfilter (232) eine Vielzahl von Poren darin definiert und das Bauteil der Entlüftungsadaptermontage mindestens teilweise aus Polycarbonat gebildet ist; und
Ultraschallverschweißen des gesinterten porösen Polytetrafluorethylenfilters (232) mit dem Bauteil der Entlüftungsadaptermontage, sodass mindestens ein Teil des Polycarbonats des Bauteils der Entlüftungsadaptermontage weich wird und in einige der Vielzahl von Poren des gesinterten porösen Polytetrafluorethylenfilters strömt und diese mindestens teilweise einnimmt.

13. Verfahren nach Anspruch 12, wobei das Ultraschallverschweißen des gesinterten porösen Polytetrafluorethylenfilters (232) mit dem Bauteil ein Ultraschallverschweißen des gesinterten porösen Polytetrafluorethylenfilters mit dem Bauteil mit einer Frequenz von etwa 10 kHz bis etwa 60 kHz und/oder einem Schweißabstand von etwa 0,001 Zoll (0,03 mm) bis etwa 0,01 Zoll (0,3 mm) und/oder einer Schweißzeit von etwa 0,075 Sekunden bis etwa 0,3 Sekunden und/oder einer Haltezeit von etwa 0,5 Sekunden bis etwa 2 Sekunden und/oder einem Manometerdruck von etwa 5 psi (34 kPa) bis etwa 20 psi (140 kPa) beinhaltet.

14. Verfahren nach Anspruch 12, wobei das Bauteil ein nichtporöses Polycarbonatbauteil beinhaltet.

## Revendications

1. Ensemble d'adaptateur d'évent (102), comprenant :
un corps de vidange (222) définissant une ouverture d'évent (228), une ouverture proximale (236), une ouverture distale (238) espacée de l'ouverture proximale (236) et une lumière de vidange (225) s'étendant entre les ouvertures proximale (236) et distale (238), la lumière de vidange étant en communication fluidique avec l'ouverture d'évent (228) ; et
un filtre poreux fritté en polytétrafluoroéthylène (232) disposé en communication fluidique avec l'ouverture d'évent (228) du corps de vidange, le filtre poreux fritté en polytétrafluoroéthylène (232) définissant une pluralité de pores en son sein,
le filtre poreux fritté en polytétrafluoroéthylène (232) étant soudé directement à un composant de l'ensemble d'adaptateur d'évent (102), le composant étant formé au moins partiellement à partir de polycarbonate ;
dans lequel au moins certains de la pluralité de pores du filtre poreux fritté en polytétrafluoroéthylène (232) sont au moins partiellement occupés par le polycarbonate du composant de l'ensemble d'adaptateur d'évent (102).

2. Ensemble d'adaptateur d'évent selon la revendication 1 dans lequel le composant de l'ensemble d'adaptateur d'évent inclut le corps de vidange (222), facultativement dans lequel le corps de vidange (222) inclut un orifice d'échantillonnage en communication fluidique avec la lumière de vidange (225).

3. Ensemble d'adaptateur d'évent selon la revendication 1 dans lequel le composant de l'ensemble d'adaptateur d'évent inclut un élément de rétention (230) qui définit une ou plusieurs ouvertures (252) en son sein, les une ou plusieurs ouvertures (252) étant couplées de manière fluide au filtre poreux fritté en polytétrafluoroéthylène (232).

4. Ensemble d'adaptateur d'évent selon la revendication 1, comprenant en outre un régulateur d'écoulement (512) disposé au moins partiellement dans la lumière de vidange (225) du corps de vidange (222), le régulateur d'écoulement (512) étant configuré pour réguler un écoulement de fluide à travers la lumière de vidange (225) vers l'ouverture distale (238).

5. Ensemble d'adaptateur d'évent selon la revendication 4, dans lequel le régulateur d'écoulement inclut :
une barrière d'écoulement (578) positionnée au moins partiellement à l'intérieur de la lumière de vidange (225) ; et
un actionneur (582) couplé de manière fonctionnelle à la barrière d'écoulement (578), l'actionneur étant configuré pour déplacer la barrière d'écoulement (582) entre une première position et une seconde position ;
dans lequel la barrière d'écoulement permet à du fluide de s'écouler à travers la lumière de vidange (225) vers l'ouverture distale lorsque la barrière d'écoulement (578) est dans la première position et empêche sensiblement le fluide de s'écouler à travers la lumière de vidange (225) vers l'ouverture distale lorsque la barrière d'écoulement (578) est dans la seconde position, dans lequel facultativement le filtre poreux fritté en polytétrafluoroéthylène (232) est fixé à l'actionneur (582), l'actionneur définit au moins un canal d'écoulement (586) en son sein qui est couplé au filtre poreux fritté en polytétrafluoroéthylène.

6. Ensemble d'adaptateur d'évent selon la revendication 1, comprenant en outre un capuchon (234) configuré pour être fixé de manière amovible au corps de vidange (222) et présentant une taille et une forme configurées pour couvrir au moins une partie de l'ouverture d'évent (228).

7. Ensemble d'adaptateur d'évent selon la revendication 1 dans lequel le filtre poreux fritté en polytétrafluoroéthylène (232) inclut un film poreux fritté en polytétrafluoroéthylène.

8. Système de poche de vidange urinaire (100), comprenant :
une poche de vidange (104) incluant une entrée (114) et configurée pour stocker au moins un fluide en son sein ;
un cathéter (106) ;
au moins un tube de vidange (108) couplant de manière fluidique l'entrée (114) de la poche de vidange (104) au cathéter (106) ; et
l'ensemble d'adaptateur d'évent (102) selon l'une quelconque des revendications 1 à 7, l'ensemble d'adaptateur d'évent couplé au au moins un tube de vidange (108) entre la poche de vidange (104) et le cathéter (106).

9. Système de poche de vidange urinaire selon la revendication 8, dans lequel le au moins un tube de vidange inclut :
un premier tube de vidange (108a) s'étendant de l'entrée (114) de la poche de vidange à l'ensemble d'adaptateur d'évent (102) ; et
un second tube de vidange (108b) s'étendant de l'adaptateur d'évent vers le cathéter.

10. Système de poche de vidange urinaire selon la revendication 8 comprenant l'ensemble d'adaptateur d'évent selon la revendication 5, dans lequel la barrière d'écoulement est positionnée entre une partie de la lumière de vidange qui est en communication fluidique avec le filtre et l'orifice d'échantillonnage lorsque la barrière d'écoulement est dans la seconde position.

11. Système de poche de vidange urinaire selon la revendication 8, dans lequel l'orifice d'échantillonnage est positionné en amont du régulateur d'écoulement.

12. Procédé de soudage par ultrasons d'un filtre poreux fritté en polytétrafluoroéthylène (232) à un composant d'un ensemble d'adaptateur d'évent (102), le procédé comprenant les étapes consistant à :
positionner le filtre poreux fritté en polytétrafluoroéthylène (232) adjacent au composant de l'ensemble d'adaptateur d'évent (102), le filtre poreux fritté en polytétrafluoroéthylène (232) définissant une pluralité de pores en son sein et le composant de l'ensemble d'adaptateur d'évent étant au moins partiellement formé de polycarbonate ; et
souder par ultrasons le filtre poreux fritté en polytétrafluoroéthylène (232) au composant de l'ensemble d'adaptateur d'évent de telle sorte qu'au moins une partie du polycarbonate du composant de l'ensemble d'adaptateur d'évent se ramollit et s'écoule dans et occupe au moins partiellement une partie de la pluralité de pores du filtre poreux fritté en polytétrafluoroéthylène.

13. Procédé selon la revendication 12, dans lequel un soudage par ultrasons du filtre poreux fritté en polytétrafluoroéthylène (232) au composant inclut un soudage par ultrasons du filtre poreux fritté en polytétrafluoroéthylène au composant à une fréquence d'environ 10 kHz à environ 60 kHz et/ou à une distance de soudage d'environ 0,001 pouce (0,03 mm) à environ 0,01 pouce (0,3 mm) et/ou à un temps de soudage d'environ 0,075 seconde à environ 0,3 seconde et/ou à un temps de maintien d'environ 0,5 seconde à environ 2 secondes et/ou à une pression manométrique d'environ 5 psi (34 kPa) à environ 20 psi (140 kPa).

14. Procédé selon la revendication 12, dans lequel le composant inclut un composant en polycarbonate non poreux.
